# EUROPEAN PATENT APPLICATION

(11) **EP 3 097 943 A1**
(43) Date of publication of application: **30.11.2016**
(21) Application number: 15740221.5
(22) Date of filing: 22.01.2015
(51) Int. Cl.: A61M 37/00

(54) **MICRONEEDLE UNIT**

(30) Priority: 24.01.2014 JP 2014011523
(71) Applicant: Toppan Printing Co., Ltd., Tokyo 110-0016 (JP)
(72) Inventor: KATO, Hiroyuki, Tokyo 110-0016 (JP); SUZUKI, Toshiyuki, Tokyo 110-0016 (JP)
(74) Representative: TBK
(86) International application number: PCT/JP2015/051732
(87) International publication number: WO 2015/111672

(57) **Abstract**

A microneedle unit including: a microneedle having a facing surface that faces a puncture target which is to be pierced by the microneedle and an adhering surface which is a surface opposite to the facing surface, the microneedle having one or more projections on the facing surface; a recessed container that houses the microneedle; a cover formed in a plate shape and is connected to the recessed container to close an opening of the recessed container, the cover extending from the adhering surface to an area surrounding the adhering surface; and an adhering section that adheres the cover and the adhering surface. The recessed container is configured to be released from the cover in a state where the microneedle is adhered to the cover via the adhering section when receiving a force in a direction separating from the cover, and the adhering section includes a first contact surface that is in contact with the cover and a second contact surface that is in contact with the adhering surface and extends from the adhering surface to an area surrounding the adhering surface so that the second contact surface comes into contact with a portion of the puncture target located in an area surrounding the facing surface when the projections pierce the puncture target.

## Description

### [Technical Field]

The technique of the present disclosure relates to microneedles and microneedle units having a container that houses the microneedle.

### [Background Art]

Percutaneous absorption methods include administration of substances such as drugs into an administration target such as a human in a painless manner so as to deliver substances into the body through the skin.

In percutaneous absorption methods, it has been proposed to administer drugs into the skin by piercing a microneedle into the skin. The microneedle includes a projection with a size that does not provoke pain to the administration target, and the projection is configured to penetrate through the stratum corneum, which is a barrier of the skin. As the drug is absorbed into the skin through a hole created in the skin by the microneedle, the drug is percutaneously absorbed.

The projection of the microneedle is covered by a cover that protects the projection before the microneedle is used by a user, for example, as described in PTL 1. The cover is adhered to a base body that supports the projection by an adhesive.

### [Citation List]

### [Patent Literature]

[PTL 1] WO 2008/020632 A

### [Summary of the Invention]

### [Technical Problem]

The user removes the cover from the microneedle when using the microneedle. Since the cover is directly adhered to the base body of the microneedle, the base body of the microneedle may deform depending on the way the user removes the cover. This may cause the projection of the microneedle to be touched by the user's finger or the cover. Since the projection of the microneedle is formed to have a size that does not provoke pain to the puncture target, there is a risk that the tip of the projection is bent in a direction which is not suitable for piercing into the target if the microneedle is touched by the user's finger or the cover before it is pierced into the skin of the puncture target. Thus, handling of the microneedle is difficult for the user when piercing it into the skin of the puncture target. Accordingly, there is need of providing microneedles configured to protect the projection from being touched by the user's finger or the cover.

The technique of the present disclosure has an object of providing a microneedle unit having improved handling properties in piercing of the microneedle into a puncture target.

### [Solution to Problem]

In one aspect of the technique of the present disclosure, a microneedle unit includes: a microneedle having a facing surface that faces a puncture target which is to be pierced by the microneedle and an adhering surface which is a surface opposite to the facing surface, the microneedle having one or more projections on the facing surface; a recessed container that houses the microneedle, the recessed container including an opening that surrounds the microneedle; a cover formed in a plate shape and is connected to the recessed container to close the opening, the cover extending from the adhering surface to an area surrounding the adhering surface; and an adhering section located between the cover and the adhering surface to adhere the cover and the adhering surface. The recessed container is configured to be released from the cover in a state where the microneedle is adhered to the cover via the adhering section when receiving a force in a direction separating from the cover, and the adhering section includes a first contact surface that is in contact with the cover and a second contact surface that is in contact with the adhering surface and extends from the adhering surface to an area surrounding the adhering surface so that the second contact surface comes into contact with a portion of the puncture target located in an area surrounding the facing surface when the projections pierce the puncture target.

According to one aspect of the microneedle unit of the technique of the present disclosure, the projections are pierced into the puncture target when the cover adhered to the microneedle is pressed against the puncture target. Accordingly, when the microneedle is pierced into the puncture target, the microneedle is less likely to be touched by the user. As a result, the user can handle the microneedle unit with ease when piercing the microneedle into the puncture target.

In another aspect of the microneedle unit of the technique of the present disclosure, it is preferable that the adhering section is configured such that, while the projections pierce the puncture target, when a portion of the second contact surface located in the area surrounding the adhering surface is adhered to the puncture target and the cover receives a force in the direction separating from the target, the first contact surface is peeled from the cover.

According to another aspect of the microneedle unit of the technique of the present disclosure, after the projections are pierced into the puncture target, the microneedle is peeled from the cover. Accordingly, the user can handle the microneedle with ease when piercing the microneedle into the puncture target.

In another aspect of the microneedle unit of the technique of the present disclosure, it is preferable that an adhesive strength of the second contact surface to the puncture target is larger than a force necessary for increasing a distance between the second contact surface and the cover.

According to another aspect of the microneedle unit of the technique of the present disclosure, since an adhesive strength of the second contact surface to the puncture target is larger than a force necessary for increasing a distance between the second contact surface and the cover, the cover is easily peeled from the microneedle.

In another aspect of the microneedle unit of the technique of the present disclosure, the cover includes a third contact surface that comes into contact with a portion of the puncture target located in the area surrounding the adhering section when the projections pierce the puncture target. It is preferable that at least part of the third contact surface has adhesiveness to the puncture target and an adhesive strength of the third contact surface to the puncture target is smaller than an adhesive strength of a portion of the second contact surface located in the area surrounding the adhering surface to the puncture target.

According to another aspect of the microneedle unit of the technique of the present disclosure, in the configuration in which at least part of the third contact surface has adhesive properties to the puncture target, when the microneedle is inserted into the puncture target, the third contact surface together with part of the second contact surface of the adhering section adheres to the puncture target. Accordingly, the microneedle is inserted into the puncture target while the position of the microneedle to the puncture target is positioned by the third contact surface. Therefore, the microneedle is easily inserted into the puncture target when a force that presses it against the puncture target is applied to the microneedle.

In another aspect of the microneedle unit of the technique of the present disclosure, it is preferable that a forming material of the first contact surface and a forming material of the second contact surface are different from each other.

According to another aspect of the microneedle unit of the technique of the present disclosure, since two the contact surfaces are made of different forming materials, the degree of freedom of the forming material of the adhering section is improved compared with the case in which the two contact surfaces are made of the same material.

In another aspect of the microneedle of the technique of the present disclosure, it is preferable that the cover includes a plurality of stepped portions on at least part of a portion that is in contact with the first contact surface.

According to another aspect of the microneedle unit of the technique of the present disclosure, a simple configuration of forming the stepped portions on the cover allows an adhesive strength of the first contact surface to the cover to be smaller than an adhesive strength of a portion of the second contact surface located in the area surrounding the adhering surface to the puncture target.

In another aspect of the microneedle unit of the technique of the present disclosure, it is preferable that the adhering section includes a first portion having the first contact surface, a second portion having the second contact surface, and a third portion disposed between the first portion and the second portion in a direction in which the microneedle and the puncture target face each other, the third portion having rigidity higher than the second portion.

According to another aspect of the microneedle unit of the technique of the present disclosure, in the direction in which the microneedle faces the puncture target, the third portion having rigidity higher than that of the second portion which is in contact with the microneedle is disposed at a position close to the first contact surface, which is configured to be peeled from the cover. Accordingly, when the cover is peeled from the microneedle, deformation of the second portion can be prevented since the adhering section includes the third portion. As a result, the microneedle and thus the projections of the microneedle are resistant to deformation, and the microneedle pierced in the puncture target is prevented from easily dropping off from the puncture target.

In another aspect of the microneedle unit of the technique of the present disclosure, the recessed container further includes a flange section that extends outward from an edge of the opening. It is preferable that the cover further includes a flange facing section that faces the flange section, and the flange section and the flange facing section are connected to each other.

According to another aspect of the microneedle unit of the technique of the present disclosure, the user can hold the flange section and the flange facing section and pull the flange section in the direction separating from the flange facing section so as to release connection between the recessed container and the cover. Accordingly, the recessed container and the cover can be easily released from each other.

### [Brief Description of the Drawings]

Fig. 1 is an exploded perspective view which shows an exploded perspective configuration of an embodiment of a microneedle unit in the present disclosure.
Fig. 2 is a cross sectional view which shows a cross sectional configuration of the microneedle unit.
Fig. 3 is a cross sectional view which shows a cross sectional configuration of the microneedle unit.
Fig. 4 is an operation view which shows an operation of the microneedle unit.
Fig. 5 is an operation view which shows an operation of the microneedle unit.
Fig. 6 is an operation view which shows an operation of the microneedle unit.
Fig. 7 is an operation view which shows an operation of the microneedle unit.
Fig. 8 is an operation view which shows an operation of the microneedle in a modified example.
Fig. 9 is an operation view which shows an operation of the microneedle in a modified example.

### [Description of Embodiments]

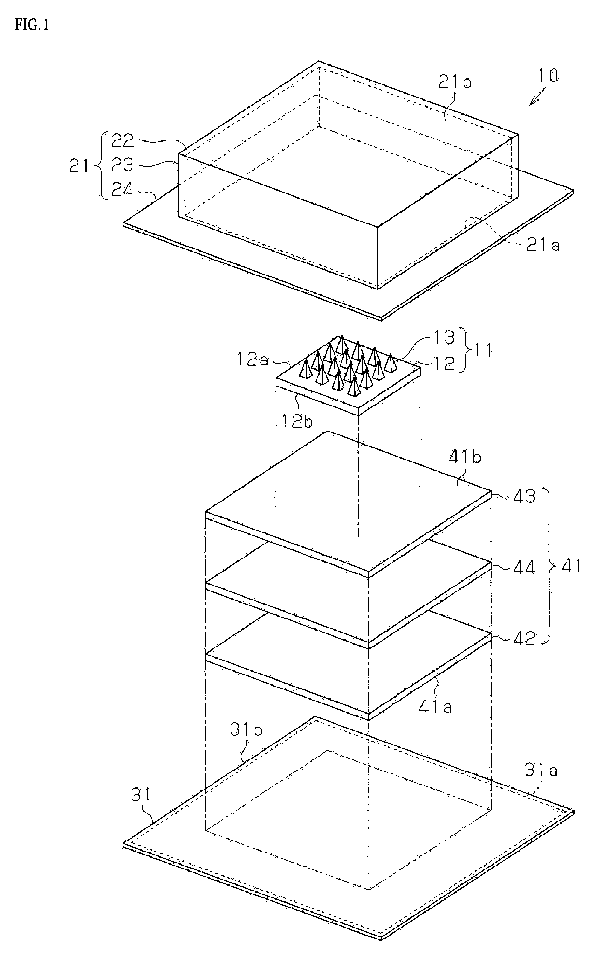

With reference to Figs. 1 to 7, an embodiment of a microneedle unit according to the present disclosure will be described. The following describes an overall configuration of a microneedle unit, a configuration of an adhering section, a configuration of a microneedle, a constituent material of the microneedle and an operation of the microneedle.

### [Overall configuration of microneedle unit]

With reference to Fig. 1, a configuration of a microneedle unit will be described.

As shown in Fig. 1, a microneedle unit 10 includes a microneedle 11, a container 21, a cover 31 and an adhering section 41. The microneedle 11 has a facing surface 12a that faces a puncture target to be pierced by the microneedle 11 and an adhering surface 12b which is a surface opposite to the facing surface 12a. The facing surface 12a is provided with one or more projections 13.

The container 21 is a recessed container that houses the microneedle 11, and includes an opening 21a that surrounds the microneedle 11. The cover 31 has a plate shape that closes the opening 21a of the container 21 and is connected to the container 21. The cover 31 extends from the adhering surface 12b of the base body 12 to an area surrounding the adhering surface 12b so as to support the adhering surface 12b of the microneedle 11. The adhering section 41 serves to adhere the cover 31 and the adhering surface 12b of the base body 12.

When the container 21 receives a force in the direction separating from the cover 31, it is separated from the cover 31 while the microneedle 11 is adhered to the cover 31 via the adhering section 41. The adhering section 41 includes a cover contact surface 41a that is in contact with the cover 31. The adhering section 41 includes a target contact surface 41b that is in contact with the adhering surface 12b of the base body 12 and extends from the adhering surface 12b to the area surrounding the adhering surface 12b. The target contact surface 41b comes into contact with a portion of the puncture target located in an area surrounding the facing surface 12a of the base body 12 when the projections 13 of the microneedle 11 pierce the puncture target.

When a portion of the target contact surface 41b located in the area surrounding the adhering surface 12b of the base body 12 is adhered to the puncture target and the cover 31 receives a force in the direction separating from the puncture target, the cover contact surface 41a is peeled from the cover 31. The cover contact surface 41a is one example of the first contact surface, and the target contact surface 41b is one example of the second contact surface.

The base body 12 of the microneedle 11 has a rectangular plate shape. The base body 12 may have a disc shape or a polygonal plate shape other than a rectangle. The facing surface 12a of the base body 12 only needs to have an area not smaller than an area that can be provided with at least one projection 13. The facing surface 12a of the base body 12 has a flat surface. The facing surface 12a may have a curved surface or may have both a flat surface and a curved surface. The adhering surface 12b of the base body 12 has a flat surface. The adhering surface 12b may have a curved surface or may have both a flat surface and a curved surface.

A plurality of projections 13 are disposed on the facing surface 12a of the base body 12. The plurality of projections 13 are arranged in a regular manner, for example, in a matrix pattern. The plurality of projections 13 may also be provided irregularly. If the plurality of projections 13 are arranged regularly, they may be arranged in a close-packed pattern or in a concentric pattern.

The respective projections 13, for example, each having a quadrangular pyramid shape, extend in one direction, which is an extending direction. Each projection 13 may have any shape as long as it can pierce the puncture target. The shape is not limited to a quadrangular pyramid, and may be, for example, another pyramid shape, frustum, column or blade shape. When the projection 13 has another pyramid shape, the projection 13 may have a conical shape or a polygonal pyramid shape other than a quadrangular pyramid shape. When the projection 13 has a frustum shape, the projection 13 may have a conical frustum shape or a polygonal frustum shape. When the projection 13 has a columnar shape, each projection 13 may have a cylindrical shape or a polygonal columnar shape.

The projections 13 may have two or more different shapes in the extending direction, for example, two or more of the above-mentioned pyramid shape, frustum shape and columnar shape. The outer peripheral surface of the projections 13 may have a twist or a step. For the purpose of facilitating puncturing the projections 13 into a puncture target, it is preferable that at least a tip of the projection 13 has a pyramid shape.

Further, the plurality of projections 13 may have mutually different shapes, or alternatively, the plurality of projections 13 may have mutually different lengths in the extending direction. For the purpose of facilitating manufacturing of the plurality of projections 13, it is preferable that the plurality of projections 13 have an identical shape. Further, the plurality of projections 13 may extend in directions crossing each other. When the plurality of projections 13 extend in directions crossing each other, the tips of the adjacent projections 13 are not in contact with each other. Further, it is preferable that the tips of the plurality of projections 13 are located on the same plane.

Alternatively, the base body 12 may have one projection 13 on the facing surface 12a. In this case, the projection 13 may be disposed at the center of the facing surface 12a or may be disposed at any position other than the center.

The container 21, which is one example of the recessed container, includes a cylindrical body 23 having a quadrangular cylindrical shape and a bottom 22 having a rectangular plate shape that closes one of both ends of the cylindrical body 23. The container 21 may have a hemispherical shape, semielliptical shape or a polygonal cylindrical shape other than a quadrangular cylindrical shape. The bottom 22 may have a curved plate shape that is recessed toward the opening 21a or a curved plate shape that bulges in a direction away from the opening 21a.

The microneedle 11 is disposed at a position that faces the center of the bottom 22 of the container 21. The microneedle 11 may be disposed at a position that faces a region other than the center of the bottom 22. The length of the container 21 in the extending direction is larger than the length of the microneedle 11 in the extending direction.

The container 21 includes a flange section 24, which extends from the entire edge of the opening 21a to the outside of a housing space 21b that is surrounded by the inner surface of the container 21. That is, the flange section 24 extends from the edge of the opening 21a to the outside of the edge of the opening 21a. The flange section 24 may be disposed on part of the edge of the opening 21a.

The cover 31 has a rectangular plate shape. The cover 31 may have a disc shape or a polygonal plate shape other than a rectangle. The cover 31 has an area larger than that of a portion surrounded by the edge of the opening 21a of the container 21. The microneedle 11 is disposed at the center of the cover 31. The microneedle 11 may be disposed at a position other than the center of the cover 31.

The container 21 and the cover 31 may be connected to each other by adhesion using an adhesive, or may be connected to each other by heat seal adhesion. Alternatively, the container 21 and the cover 31 may each have a fitting section so that the container 21 and the cover 31 are connected to each other by the fitting sections of the container 21 and the cover 31 fitting in each other. The container 21 can be released from the cover 31 when receiving a force in the direction separating from the cover 31.

The cover 31 includes a flange facing section 31a that faces the flange section 24 on a surface that faces the container 21. An area of the surface of the cover 31 which faces the container 21 is preferably not smaller than an area surrounded by the outer edge of the flange section 24.

The flange facing section 31a and the flange section 24 are mutually connected. A user can hold the flange section 24 and the flange facing section 31a and pull the flange section 24 in the direction separating from the flange facing section 31a so as to release connection between the container 21 and the cover 31. Accordingly, the container 21 and the cover 31 can be easily released from each other.

The cover 31 includes an outer peripheral contact surface 31b. When the projections 13 of the microneedle 11 pierce the puncture target, the outer peripheral contact surface 31b comes into contact with a portion of the puncture target located in the area surrounding the adhering section 41. The outer peripheral contact surface 31b is one example of the third contact surface.

At least part of the outer peripheral contact surface 31b has adhesiveness to the puncture target. The cover 31 may be made of a material having adhesiveness to the puncture target. Alternatively, a member having adhesiveness to the puncture target may be disposed on the outer peripheral contact surface 31b.

The outer peripheral contact surface 31b has adhesive strength to the puncture target which is smaller than that of a portion of the target contact surface 41b located in the area surrounding the adhering surface 12b of the base body 12. According to the configuration in which at least part of the outer peripheral contact surface 31b has adhesiveness to the puncture target, when the microneedle 11 is pierced into the puncture target, the outer peripheral contact surface 31b together with part of the adhering section 41 of the microneedle 11 adheres to the puncture target. Accordingly, the microneedle 11 is pierced into the puncture target while the position of the microneedle 11 to the puncture target is positioned by the outer peripheral contact surface 31b. Therefore, when a force in the direction toward the puncture target is applied to the microneedle 11, piercing by the microneedle 11 into the puncture target is facilitated.

The flange facing section 31a of the cover 31 and a portion of the outer peripheral contact surface 31b having adhesiveness may overlap each other. In this case, it is preferable that the portion of the outer peripheral contact surface 31b having adhesiveness also has adhesiveness to the container 21 and is located on the entire outer peripheral contact surface 31b. Alternatively, an adhering member that adheres the outer peripheral contact surface 31b and the flange section 24 may be provided between the outer peripheral contact surface 31b and the flange section 24. In this case, the adhering member that adheres the outer peripheral contact surface 31b and the flange section 24 may be a member that is peeled from the outer peripheral contact surface 31b and remain on the flange section 24 when the cover 31 and the container 21 are released from each other. Further, the adhering member may be a member that is peeled from the flange section 24 and remain on the outer peripheral contact surface 31b when the cover 31 and the container 21 are released from each other.

Alternatively, the flange facing section 31a of the cover 31 and a portion of the outer peripheral contact surface 31b having adhesiveness to the puncture target may not necessarily overlap each other in the cover 31. That is, the cover 31 may separately include the flange facing section 31a and the portion of the outer peripheral contact surface 31b having adhesiveness to the puncture target.

### [Configuration of adhering section]

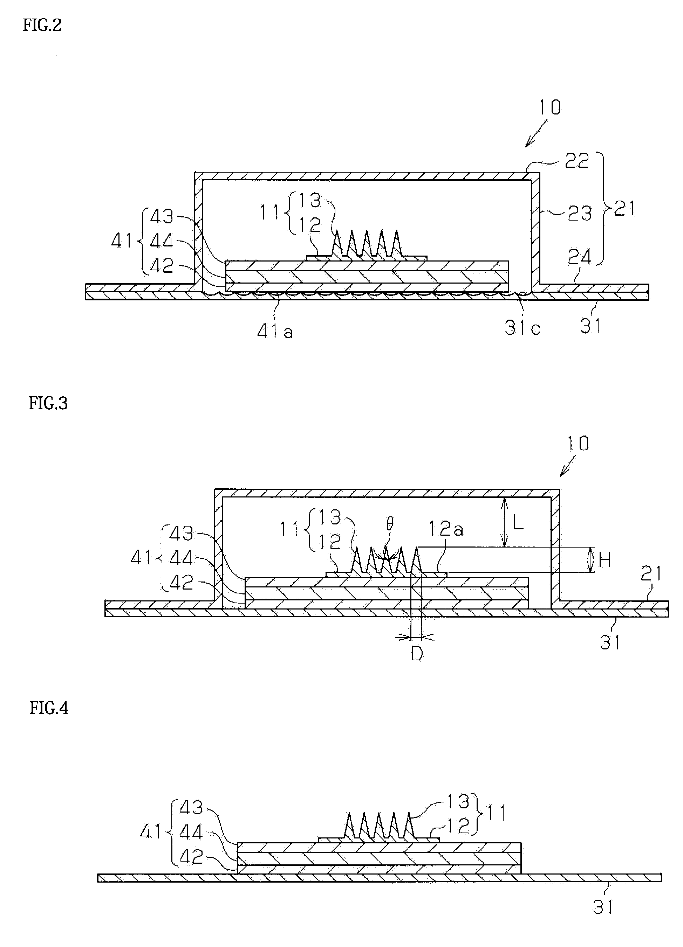

With reference to Figs. 1 and 2, the adhering section 41 of the microneedle unit 10 will be described in detail.

As shown in Fig. 1, the adhering section 41 has, for example, a plate shape. In the adhering section 41, the cover contact surface 41a has adhesive strength to the cover 31, which is referred to as cover adhesive strength. Further, a portion of the target contact surface 41b located in the area surrounding the adhering surface 12b has adhesive strength to the puncture target, which is referred to as target adhesive strength.

When the projections 13 of the microneedle 11 are pierced into the puncture target, the cover adhesive strength is preferably smaller than the target adhesive strength. Further, the cover adhesive strength is an adhesive strength exhibited by the entire surface area of the cover contact surface 41a which is in contact with the cover 31. The target adhesive strength is an adhesive strength exhibited by the entire surface area of a portion of the target contact surface 41b located in the area surrounding the adhering surface 12b.

In the adhering section 41, the cover adhesive strength and the target adhesive strength are different due to the configuration described below. That is, two adhesive strengths may be different due to the different adhesion targets to which the surfaces each adhere, the difference in forming material of each surface, or the difference in the amount of contact surface area between each surface and each adhesion target, or combinations thereof.

The adhering section 41 is made up of a plurality of different layers laminated in the extending direction, and the adhering section 41 serves as a laminate that adheres the cover 31 and the adhering surface 12b of the base body 12. The adhering section 41 includes a cover contact section 42 having the cover contact surface 41a that is in contact with the cover 31, a target contact section 43 having the target contact surface 41b that is in contact with the adhering surface 12b of the base body 12 and the puncture target. The cover contact section 42 and the target contact section 43 each have a layer shape. The cover contact section 42 is one example of the first portion, and the target contact section 43 is one example of the second portion.

The target contact section 43 extends from the adhering surface 12b of the base body 12 to the area surrounding the adhering surface 12b. When the projections 13 pierce the puncture target, a portion of the puncture target located in the area surrounding the facing surface 12a comes into contact with the target contact surface 41b of the target contact section 43. It is preferable that the forming material of the cover contact section 42 and the forming material of the target contact section 43 are different from each other. The target contact section 43 has adhesiveness on the target contact surface 41b so as to exhibit the adhesive strength to the puncture target.

The adhering section 41 includes a portion located on the cover contact surface 41a and exhibiting the adhesive strength to the cover 31 and a portion located on the target contact surface 41b and exhibiting the adhesive strength between the adhering section 41 and the puncture target, which are independent of each other. Accordingly, compared with the adhering section 41 formed as a single layer, the degree of freedom of the forming material of the adhering section 41 is improved.

Specifically, in the adhering section 41 having the cover contact section 42 and the target contact section 43, use of the following configuration allows the cover adhesive strength to be smaller than the target adhesive strength.
(A) As shown in Fig. 2, on the inner surface of the cover 31 that is in contact with the cover contact surface 41a, an uneven area 31c is formed, for example, by providing an embossed pattern in a portion which does not face the flange section 24 of the container 21, that is, a portion of the inner surface which is in contact with the cover contact surface 41a of the adhering section 41. This contributes to decreasing the contact area between the cover 31 and the adhering section 41 to be smaller than the contact area between the puncture target and the adhering section 41.
(B) The inner surface of the cover 31 which is in contact with the cover contact surface 41a is coated with, for example, silicone material or a fluoride material.
(C) Materials having different adhesive strengths are selected as the forming material of the cover contact section 42 and the forming material of the target contact section 43. Particularly, it is preferable that the forming material of the cover contact section 42 has an adhesive strength per unit area smaller than that of the forming material of the target contact section 43. For example, the forming material of the cover contact section 42 may be an adhesive having easy-peel properties, in other words, easy-separation properties, or an adhesive having re-adhesion properties. Specifically, an adhesive material such as polyurethane material, acrylic material, polyvinyl chloride material, polyvinylidene chloride material, vinyl acetate material, and silicone material can be used. In particular, an adhesive of easy-separation grade (easy-peel type) such as an easy-separation type silicone adhesive is preferable.

According to the above (A), the adhesive strength per unit area between the cover contact surface 41a and the cover 31 may be larger than the adhesive strength per unit area between the portion of the target contact surface 41b which comes into contact with the puncture target and the puncture target. In this configuration as well, the cover adhesive strength is smaller than the target adhesive strength.

According to the above (B), the adhesive strength per unit area between the cover contact surface 41a and the cover 31 may be larger than the adhesive strength per unit area between the portion of the target contact surface 41b which comes into contact with the puncture target and the puncture target. In this configuration as well, the adhesive strength between the cover contact surface 41a and the cover 31 is smaller than the adhesive strength between the portion of the target contact surface 41b which comes into contact with the puncture target and the puncture target since the treatment described in (B) is applied. Furthermore, when the above (A) or (B) is used, the forming material of the cover contact section 42 and the forming material of the target contact section 43 may be an adhesive material such as a polyurethane material, acrylic material, polyvinyl chloride material, polyvinylidene chloride material, vinyl acetate material and silicone material.

In order to achieve the above (C), the forming material of the target contact section 43 may be an adhesive material such as a polyurethane material, acrylic material, polyvinyl chloride material, polyvinylidene chloride material, vinyl acetate material and silicone material.

As shown in Fig. 1, the adhering section 41 further includes a support section 44 between the cover contact section 42 and the target contact section 43 in the direction in which the microneedle 11 faces the puncture target, that is, in the direction parallel with the extending direction. The support section 44 is formed in a layer shape and has rigidity higher than that of the target contact section 43. The support section 44 is one example of the third portion.

According to the configuration in which the support section 44 is disposed between the cover contact section 42 and the base body 12, the support section 44 having high rigidity can prevent deformation of the target contact section 43 when the cover 31 and the target contact section 43 are separated from each other and the cover 31 is peeled from the microneedle 11. Accordingly, the base body 12 and thus the projections 13 are prevented from being easily deformed. As a result, when the cover 31 is peeled from the microneedle 11, the projections 13 of the microneedle 11 pierced into the puncture target are prevented from being easily dropped off from the puncture target.

The support section 44 is, for example, a resin film made of a material having rigidity higher than that of the forming material of the target contact section 43. The support section 44 is, for example, a resin film made of a resin such as polyethylene, polypropylene and polyethylene terephthalate.

When the adhering section 41 includes the support section 44, use of the following configuration allows the cover adhesive strength to be smaller than the target adhesive strength.

(D) The cover contact section 42 is formed to be smaller than the support section 44 and the target contact section 43 so that the cover contact section 42 is in contact with only part of the support section 44. This contributes to decreasing the contact area between the cover 31 and the cover contact section 42.

According to the above (D), if the adhesive strength per unit area between the cover contact surface 41a and the cover 31 is larger than the adhesive strength per unit area between the portion of the target contact surface 41b which comes into contact with the puncture target and the puncture target, the adhesive strength between the cover contact surface 41a and the cover 31 can be relatively decreased. Alternatively, two or more of the above (A) to (D) can be combined.

The adhering section 41 may be formed of a single layer. In this case, the adhesive strength of the cover contact surface 41a to the cover 31 per unit area may be equal to the adhesive strength of a portion of the target contact surface 41b located in the area surrounding the adhering surface 12b to the puncture target per unit area. In this configuration as well, the cover adhesive strength is smaller than the target adhesive strength by use of at least one of the above (A) and (B).

When the adhering section 41 is formed of a single layer, the composition of the adhering section 41 may be uniform in the extending direction or may vary in the extending direction. When the adhering section 41 formed of a single composition, the forming material of the adhering section 41 is polyurethane material, acrylic material, polyvinyl chloride material, polyvinylidene chloride material, vinyl acetate material, silicone material or the like.

### [Configuration of microneedle]

With reference to Fig. 3, a configuration of the microneedle 11 will be described. Fig. 3 is a view of a cross sectional configuration of the microneedle unit 10 taken along the plane which is perpendicular to the surface of the cover 31 that is in contact with the adhering section 41 and passes through the container 21, the microneedle 11 and the adhering section 41.

As shown in Fig. 3, in the projection 13 of the microneedle 11, a height H of the projection 13 is a length in the extending direction from the facing surface 12a of the base body 12 to the tip which is a distal end of the projection 13. The projection 13 preferably has the height H suitable for creating a hole in the skin of the puncture target, and the height H of the projection 13 is preferably in the range from 10 µm to 1000 µm, inclusive.

The height H of the projection 13 is preferably designed depending on where in the skin that the bottom of the hole to be formed in the puncture target is located. When the bottom of the hole is designed to be located in the stratum corneum, the height H of the projection 13 is preferably in the range from 10 µm to 300 µm, inclusive, more preferably in the range from 30 µm to 200 µm, inclusive.

When the bottom of the hole is designed to pass through the stratum corneum of the skin and to be located at a position not reaching the nerve plexus, the height H of the projection 13 is preferably in the range from 200 µm to 700 µm, inclusive, more preferably in the range from 200 µm to 500 µm, inclusive. When the bottom of the hole is designed to pass through the stratum corneum of the skin and to be located at a position not reaching the nerve plexus, the height H of the projection 13 is further preferably in the range from 200 µm to 300 µm, inclusive.

When the bottom of the hole is designed to be located at a position reaching the dermis, the height H of the projection 13 is preferably in the range from 200 µm to 500 µm, inclusive. When the bottom of the hole is designed to be located at a position reaching the epidermis, the height H of the projection 13 is preferably in the range from 200 µm to 300 µm, inclusive.

In the projection 13, the maximum length in the direction perpendicular to the extending direction and along the facing surface 12a is a width D, and the width D of the projection 13 is preferably in the range from 1 µm to 300 µm, inclusive. For example, when the projection 13 has a regular quadrangular pyramid shape or a square column shape, the proximal end which is different from the distal end of the projection 13 defines a square shape on the facing surface 12a. A diagonal length of the square area defined by the proximal end of the projection 13 is a width D of the projection 13. Further, for example, when the projection 13 has a conical or columnar shape, the proximal end of the projection 13 defines a circular shape on the facing surface 12a. The diameter of the circular shape defined by the proximal end of the projection 13 is the width D of the projection 13.

In the projection 13, an aspect ratio A is the ratio of the height H to the width D (A = H/D). The aspect ratio A of the projection 13 is preferably in the range from 1 to 10, inclusive.

When the projection 13 has a pyramid shape at least on the distal end, the angle of the distal end of the projection 13 is a distal end angle θ. The distal end angle θ is a maximum angle of the projection 13 in the cross section taken along the plane perpendicular to the facing surface 12a of the base body 12. For example, when the projection 13 has a regular quadrangular pyramid shape, the distal end angle θ of the projection 13 is an apex angle of an isosceles triangle having the proximal end of the projection 13 as a base and the distal end of the projection 13 as an apex.

When the projection 13 has a pyramid shape at least on the distal end and the bottom of the hole is designed to be located at a position that passes through the stratum corneum, the distal end angle θ is preferably in the range from 5 degrees to 30 degrees, inclusive, more preferably in the range from 10 degrees to 20 degrees, inclusive.

In the microneedle unit 10, a distance between the distal end of the projection 13 and a portion of the container 21 which faces the distal end of the projection 13 is a spaced distance L. The spaced distance L is preferably in the range from 0.1 mm to 10 mm, inclusive. In the configuration having the spaced distance L of not smaller than 0.1 mm, the distal end of the projection 13 can be prevented from coming into contact with the container 21 during shipping of the microneedle unit 10, thereby preventing the deformation or damage of the distal end of the projection 13. Further, in the configuration having the spaced distance L of not larger than 10 mm, a space necessary for shipping and storage of the microneedle unit 10 can be prevented from increasing in size during shipping and storage of the microneedle unit 10.

### [Constituent material of microneedle unit]

The forming material of the microneedle 11 is preferably a biocompatible material, that is, a material which functions as the microneedle 11 but does not adversely affect the puncture target to which the microneedle 11 is applied. When the forming material of the microneedle 11 is a biocompatible material, the forming material is silicon, metal, resin or the like. When the forming material of the microneedle 11 is a metal, the forming material is stainless steel, titanium, mangan or the like. When the forming material of the microneedle 11 is a resin, the forming material is medical grade silicone, polylactic acid, polyglycolic acid, polycarbonate cyclic olefin copolymer or the like.

The forming material of the microneedle 11 may be a material having biocompatibility and soluble in liquid administered to the puncture target by the microneedle unit 10 or liquid administered to the puncture target from outside. When the forming material is a material that dissolves in liquid, the forming material is, for example, a water soluble polymer. The water soluble polymer is, for example, alginates, curdlan, chitin, chitosan, glucomannan, polymalic acid, collagen, collagen peptide, hydroxypropyl cellulose, hydroxypropylmethyl cellulose, carboxymethyl cellulose, gelatin, chitosan succinamide, trimethyl chitosan, oligo chitosan, oligo chitin, ethylene glycol chitosan, ethylene glycol chitin or the like.

In the microneedle 11, the forming material of the base body 12 and the forming material of the projection 13 may be the same or different from each other. When the microneedle 11 includes the plurality of projections 13, the forming materials of the projections 13 may be the same or different from each other.

A method for manufacturing the microneedle 11 may be one of a variety of known methods. When the forming material of the microneedle 11 is a resin, a method for manufacturing the microneedle 11 may be one of the injection molding method, extrusion molding method, imprint method, hot embossing method, casting method and the like.

When the forming material of the microneedle 11 is silicon or metal, a method for manufacturing the microneedle 11 may be a machining method such as cutting, etching method and the like.

Regardless of whether the forming material of the microneedle 11 is any of the above materials, a method for manufacturing the microneedle 11 may be a reproducing method using an intaglio plate as an original plate of the microneedle 11. The intaglio plate is manufactured by, for example, a plating method or a molding method using a resin.

The forming material of the container 21 is preferably a flexible material, and the forming material of the container 21 is a resin such as polyethylene, polypropylene, polyethylene terephthalate or the like.

The forming material of the cover 31 is preferably a flexible material. For example, similarly to the container 21, the forming material of the cover 31 is a resin such as polyethylene, polypropylene, polyethylene terephthalate or the like.

The microneedle unit 10 may include a liquid drug administered into the skin or a liquid drug dissolved in a predetermined solvent. In this case, the microneedle unit 10 preferably includes a drug housing space in the cover 31, which is different from the housing space of the microneedle 11, so that the drug is held in the drug housing space. The drug housing space may have any configuration as long as it allows the drug to be supplied to the puncture target when the projection 13 of the microneedle 11 is pierced into the puncture target.

The drug administered into the skin is pharmacologically active agents, cosmetic composition or the like. When the drug is a pharmacologically active agent, the drug is appropriately selected depending on the user's application. When the drug is a pharmacologically active agent, the drug is, for example, vaccines such as influenza vaccine, pain relievers for cancer patients, insulin, biologics, gene therapy agents, injections, oral agents skin application preparations or the like.

Since the microneedle unit 10 is pierced into the skin, percutaneous administration using the microneedle unit 10 is also applied to pharmacologically active agents that are need to be subcutaneously injected in addition to the pharmacologically active agents which are conventionally used for percutaneous administration. Particularly, when an injection agent such as vaccine is administered, percutaneous administration using the microneedle unit 10 does not cause a pain during drug administration. Accordingly, percutaneous administration using the microneedle unit 10 is preferably applied to children. Further, in percutaneous administration using the microneedle unit 10, a patient does not need to take the drug orally during drug administration to the patient. Accordingly, the microneedle unit 10 is preferably applied to children who have difficulty in taking oral medication.

Cosmetic composition is a composition for use as cosmetics and beauty products. When the drug is a cosmetic composition, the drug is, for example, humectants, colorants, fragrance, physiologically active agents exhibiting cosmetic effects or the like. Physiologically active agents exhibiting cosmetic effects are, for example, substances having improvement effect on wrinkles, acne, stretch marks and the like, or substance having improvement on hair loss.

The container 21 and the cover 31 may hold injection liquid used for administration of the drug into the skin separately from the drug, and the injection liquid may be, for example, water-based solvent such as water or ethylalcohol. The injection liquid may be disposed in the container 21 while being contained in a gel material or a porous material such as a sponge.

Further, when the forming material of the projection 13 is a material that is dissolved in the injection liquid held in the cover 31, the forming material of the projection 13 may contain the above drug. In this case, it is preferable that the microneedle unit 10 is configured such that the projection 13 is not in contact with the injection liquid in the cover 31 before use of the microneedle unit 10 and the projection 13 is in contact with the injection liquid in use of the microneedle unit 10. Further, the forming material of the projection 13 may be a material dissolved in the skin of the puncture target. In this case, the liquid held in the cover 31 increases the dissolving rate of the projection 13 in the skin.

When the cover 31 holds the above liquid drug or injection liquid, the cover 31 preferably has a through hole through which the liquid is introduced from the outside into the inside of the cover 31. After the liquid is introduced into the cover 31, the through hole of the cover 31 may be closed by a closing member that is disposed inside the through hole to close the through hole. When the closing member has, for example, a cylindrical shape having two cylindrical ends, it is preferable that a portion of the closing member which is in contact with the outer surface of the cover 31 is positioned such that the end on the outer surface of the cover 31 has an area larger than the area surrounded by the through hole.

### [Operation of microneedle unit]

With reference to Figs. 4 to 7, an operation of the microneedle unit 10 will be described. The puncture target for the microneedle 11 may be, for example, a skin of humans or a skin of animals other than humans. An operation of the microneedle unit 10 will be described by means of a configuration example of the microneedle unit 10 in which the cover adhesive strength is smaller than the target adhesive strength.

As shown in Fig. 4, when a user uses the microneedle unit 10, the user releases connection between the cover 31 and the container 21 so as to remove the container 21 from the microneedle unit 10. In so doing, the microneedle 11 remains adhered to the cover 31 via the adhering section 41. Since the container 21 that covers the projections 13 of the microneedle 11 is connected to the cover 31, the microneedle 11 is less likely to be subject to a force that deforms the microneedle 11 when the container 21 is removed from the microneedle unit 10. Accordingly, the base body 12 of the microneedle 11 can be prevented from being deformed, thereby preventing the projection 13 on the base body 12 from being brought into contact with the inner surface of the container 21 due to the deformed base body 12. Accordingly, the user can handle the microneedle unit 10 with ease when piercing the microneedle 11 into the puncture target.

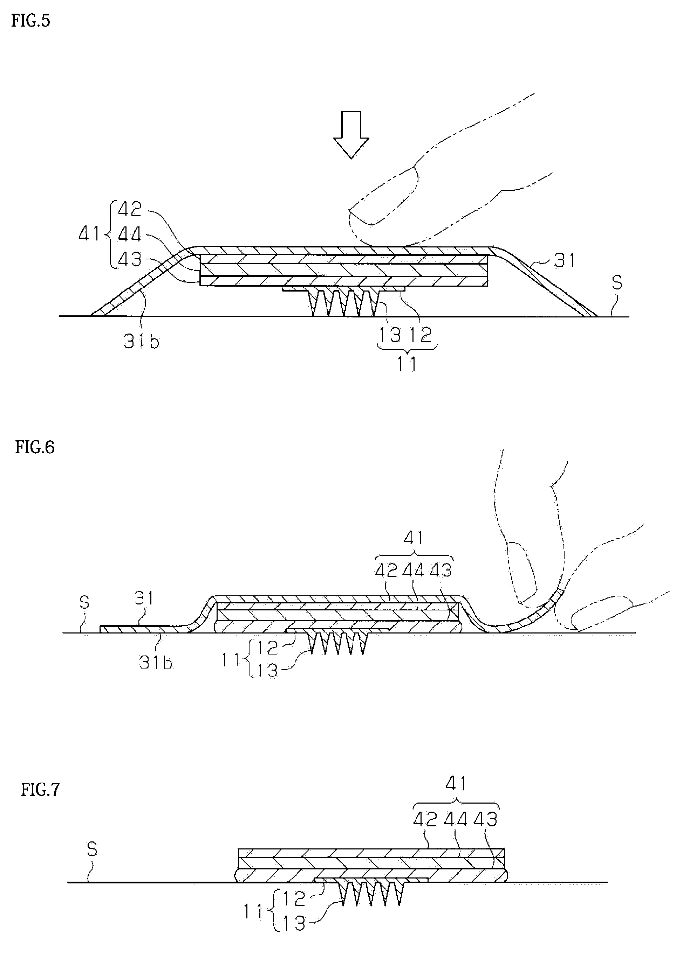

As shown in Fig. 5, for example, the user picks a portion of the cover 31 which is located around the adhering section 41 to place the microneedle 11 on a skin S, which is the puncture target.
When the microneedle 11 is placed on the skin S of the puncture target, a portion of the cover 31 which is located around the adhering section 41 is located on the surface of the skin S. Since the microneedle 11 is covered by the cover 31 when the microneedle 11 is placed on the skin S, the microneedle 11 is less likely to be touched by the user. Accordingly, the user can handle the microneedle unit 10 with ease when piercing the microneedle 11 into the puncture target.

The user pushes part of the cover 31 against the puncture target so as to pierce the projections 13 of the microneedle 11 into the skin S of the puncture target. Accordingly, holes are formed in the skin S of the puncture target by the projections 13. In the configuration in which at least part of the outer peripheral contact surface 31b of the cover 31 has adhesive properties to the puncture target, when the microneedle 11 is inserted into the puncture target, the outer peripheral contact surface 31b together with part of the adhering section 41 of the microneedle 11 adheres to the puncture target. In addition to that, since the target contact section 43 of the adhering section 41 has rigidity of an extent than can be expanded by a force applied by a user, the target contact section 43 is expanded from the adhering surface 12b of the base body 12 to the area surrounding the adhering surface 12b. Then, the target contact section 43 closely contacts with the peripheral surface of the base body 12 and the surface of the skin S.

As shown in Fig. 6, the user removes the cover 31 from the skin S. Here, the user picks a portion of the cover 31 so as to lift the cover 31 from the surface of the skin S. As a result, the cover 31 is peeled from the cover contact section 42 as a force in the direction separating from the puncture target is applied to the cover 31.

Accordingly, in the microneedle unit 10, only the microneedle 11 and the adhering section 41 are located on the surface of the skin S as shown in Fig. 7.

The microneedle 11 is covered by the cover 31 during a period from when the cover 31 is placed on the skin S of the puncture target until when the cover 31 is peeled from the microneedle 11. When the user pierces the microneedle 11 into the puncture target, the microneedle 11 is less likely deformed compared with the case in which the member that covers the microneedle 11 is not provided. Accordingly, the user can handle the microneedle unit 10 with ease when piercing the microneedle 11 into the puncture target.

When the projections 13 of the microneedle 11 is pierced into the skin S, the cover 31 that covers the microneedle 11 is located on the surface of the skin S along with the microneedle 11. Accordingly, in the case where the microneedle 11 is applied to the skin S for a long period of time, for example, in the case where the forming material of the microneedle 11 is a drug, the microneedle 11 can be prevented from being peeled without need for the user to press the microneedle 11 against the skin S.

Moreover, in the case where the microneedle 11 is used to create a hole, the user only have to pick the adhering section 41 when he/she removes the microneedle 11 from the skin S. Accordingly, the hole created in the skin S is less likely to be touched by the user.

### [Example 1]

### [Method for manufacturing microneedle]

In manufacturing of the microneedle 11, an original plate of the microneedle 11 was fabricated by micromachining of a silicon substrate. The silicon substrate was provided with 36 projections, each formed in a regular quadrangular pyramid shape with a height H of 150 µm and a base side length of 60 µm. The respective 36 projections are disposed with an interval of 1mm in a matrix of 6 columns and 6 lines.

Then, the original plate of the microneedle 11 formed of a silicon substrate was plated with a nickel film with a thickness of 500 µm. After the nickel film was formed, the original plate was etched by wet etching by using 30 wt% potassium hydroxide aqueous solution heated to 90°C to form an intaglio plate made of nickel.

After the intaglio plate was fabricated, hydroxypropyl cellulose in the liquid form was supplied to the intaglio plate. Then, hydroxypropyl cellulose was cured by heat from a heat source. The cured hydroxypropyl cellulose was peeled from the intaglio plate to obtain the microneedle 11.

### [Method for manufacturing microneedle unit]

As the container 21 of the microneedle unit 10, a container made of polyethylene terephthalate (PET) was used. The container has a quadrangular cylindrical shape with one of the cylindrical ends being closed, and includes a flange section disposed on the entire circumference of the other cylindrical end which is open. The adhering section 41 was provided as the adhering section 41 that includes the cover contact section 42, the target contact section 43, and the support section 44 which is interposed between the cover contact section 42 and the target contact section 43. The cover contact section 42 was formed of an easy-peel silicone adhesive, the target contact section 43 was formed of an acrylic adhesive, and the support section 44 was formed of polyethylene.

Further, the cover 31 of the microneedle unit 10 was formed of a film of PET having a rectangular plate shape. Then, the adhering section 41 was formed by stacking the cover contact section 42, the support section 44 and the target contact section 43, and the adhering section 41 was adhered on the inner surface of the cover 31 while the microneedle 11 was positioned at the center of the target contact section 43. Finally, the cover 31 having the adhering section 41 and the microneedle 11 and the container 21 were adhered by using an adhesive.

### [Test method and test result]

The cover 31 was pressed against a swine skin while the cover 31 was adhered to the microneedle 11 to piece the projection 13 of the microneedle 11 into the swine skin. Then, the cover 31 was released from the swine skin and removed from the swine skin. After the cover 31 was removed, the microneedle 11 and the adhering section 41 that covers the microneedle 11 were found to remain on the swine skin.

### [Example 2]

### [Method for manufacturing microneedle]

In manufacturing of the microneedle 11, an original plate of the microneedle 11 was fabricated by micromachining of a silicon substrate. The silicon substrate was provided with 36 projections, each formed in a regular quadrangular pyramid shape with a height H of 150 µm and a base side length of 60 µm. The respective 36 projections are disposed with an interval of 1mm in a matrix of 6 columns and 6 lines.

Then, the original plate of the microneedle 11 formed of a silicon substrate was plated with a nickel film with a thickness of 500 µm. After the nickel film was formed, the original plate was etched by wet etching by using 30 wt% potassium hydroxide aqueous solution heated to 90°C to form an intaglio plate made of nickel.

After the intaglio plate was fabricated, hydroxypropyl cellulose in the liquid form was supplied to the intaglio plate. Then, hydroxypropyl cellulose was cured by heat from a heat source. The cured hydroxypropyl cellulose was peeled from the intaglio plate to obtain the microneedle 11.

### [Method for manufacturing microneedle unit]

As the container 21 of the microneedle unit 10, a container made of polyethylene terephthalate (PET) was used. The container has a quadrangular cylindrical shape with one of the cylindrical ends being closed, and includes a flange section disposed on the entire circumference of the other cylindrical end which is open. The adhering section 41 was provided as the adhering section 41 which includes the cover contact section 42, the target contact section 43, and the support section 44 which is interposed between the cover contact section 42 and the target contact section 43. The cover contact section 42 was formed of an acrylic adhesive, the target contact section 43 was formed of an acrylic adhesive, and the support section 44 was formed of polyethylene.

Further, the cover 31 of the microneedle unit 10 was formed of a film of PET formed in a rectangular plate shape having an embossed dot pattern on the inner surface. Then, the adhering section 41 was formed by stacking the cover contact section 42, the support section 44 and the target contact section 43, and the adhering section 41 was adhered on the cover 31 on the inner surface having the embossed pattern while the microneedle 11 was positioned at the center of the target contact section 43. Finally, the cover 31 having the adhering section 41 and the microneedle 11 and the container 21 were adhered by using an adhesive.

### [Test method and test result]

The cover 31 was pressed against a swine skin while the cover 31 was adhered to the microneedle 11 to piece the projection 13 of the microneedle 11 into the swine skin. Then, the cover 31 was released from the swine skin and removed from the swine skin. After the cover 31 was removed, the microneedle 11 and the adhering section 41 that covers the microneedle 11 were found to remain on the swine skin.

As described above, according to the microneedle unit 10 of the embodiments, the following effect can be achieved.
(1) When the cover 31 adhered to the microneedle 11 is pressed against the puncture target, the projections 13 are pierced into the puncture target. After the projections 13 are pierced into the puncture target, the microneedle 11 is peeled from the cover 31. Accordingly, when the microneedle 11 is pierced into the puncture target, the microneedle 11 is less likely to be touched by the user. As a result, the user can handle the microneedle unit 10 with ease when piercing the microneedle 11 into the puncture target.
(2) Since the cover adhesive strength is smaller than the target adhesive strength, the cover 31 is easily peeled from the microneedle 11.
(3) In the configuration in which at least part of the outer peripheral contact surface 31b has adhesive properties to the puncture target, when the microneedle 11 is inserted into the puncture target, the outer peripheral contact surface 31b together with part of the adhering section 41 of the microneedle 11 adheres to the puncture target. Accordingly, the microneedle 11 is inserted into the puncture target while the position of the microneedle 11 in the puncture target is positioned by the outer peripheral contact surface 31b. Therefore, the microneedle 11 is easily inserted into the puncture target when a force that presses it against the puncture target is applied to the microneedle 11.
(4) Since the cover contact surface 41a and the target contact surface 41b are made of different forming materials, the degree of freedom of the forming material of the adhering section 41 is improved compared with the case in which two contact surfaces are made of the same material.
(5) In the direction in which the microneedle 11 faces the puncture target, the support section 44 having rigidity higher than that of the target contact section 43 which is in contact with the microneedle 11 is disposed at a position closer to the cover contact surface 41a, which is configured to be peeled from the cover 31, than the target contact section 43. Accordingly, when the cover 31 is peeled from the microneedle 11, deformation of the target contact section 43 can be prevented since the adhering section 41 includes the support section 44. As a result, the microneedle 11 and thus the projections 13 of the microneedle 11 are resistant to deformation, and the microneedle 11 pierced in the puncture target is prevented from easily dropping off from the puncture target.
(6) The user can hold the flange section 24 and the flange facing section 31a and pull the flange section 24 in the direction separating from the flange facing section 31a so as to release connection between the container 21 and the cover 31. Accordingly, the container 21 and the cover 31 can be easily released from each other.

Furthermore, the aforementioned embodiments can be appropriately modified as described below.

In the container 21, the flange section 24 may be omitted. In this case, the flange facing section 31a of the cover 31 may be omitted. In this configuration as well, the effect similar to the above (1) to (5) can be obtained.

The adhering section 41 may not necessarily include the support section 44. In this configuration as well, at least the effect similar to the above (1) can be obtained as long as the cover adhesive strength is smaller than the target adhesive strength in the adhering section 41.

In the adhering section 41, the forming material of the cover contact surface 41a and the forming material of the target contact surface 41b may be the same. In this configuration as well, the cover adhesive strength is smaller than the target adhesive strength by using the above (A) or (B). In this configuration, at least the effect similar to the above (1) can be obtained.

The uneven area 31c may not be necessarily formed across the entire surface of a portion of the inner surface of the cover 31 which does not face the flange section 24. The uneven area 31c may be formed only on a portion of the inner surface which is in contact with the cover contact surface 41a of the adhering section 41. Alternatively, the uneven area 31c may be formed on at least part of a portion of the inner surface which is in contact with the cover contact surface 41a of the adhering section 41. In other words, the cover adhesive strength may be decreased compared with the target adhesive strength in this configuration.

The uneven area 31c may be formed by the above embossed pattern, that is, the repeating pattern of a plurality of recesses provided on the film constituting the cover 31, or alternatively, the uneven area 31c may be formed by a plurality of recesses formed by any other method. Further, the uneven area 31c may also be formed by a plurality of bumps formed on the film constituting the cover 31. For example, the bumps may be formed by adhering a plurality of bumps formed separately from the cover 31 to the cover 31 or by removing a portion of the film other than the bumps after the separate film is adhered on the cover 31. In other words, a plurality of stepped portions may be formed on one surface of the cover 31. Accordingly, a simple configuration of forming the stepped portions on the cover 31 allows the cover adhesive strength to be smaller than the target adhesive strength. Moreover, since the above adhesive strength is achieved by the contact area between the adhering section 41 and the cover 31, the degree of freedom of the forming material of the adhering section 41 can be improved.

The adhesive strength of the cover contact section 42 to the cover 31 may be larger than the adhesive strength of the cover contact section 42 to the support section 44, and may be larger than the adhesive strength of the target contact section 43 to the support section 44. Further, a peeling adhesive strength, which is a smaller adhesive strength of the adhesive strength of the cover contact section 42 to the support section 44 and the adhesive strength of the target contact section 43 to the support section 44, is smaller than the target adhesive strength. In other words, the adhesive strength of the target contact surface 41b to the skin is larger than a force necessary for increasing a distance between the target contact surface 41b and the cover 31.

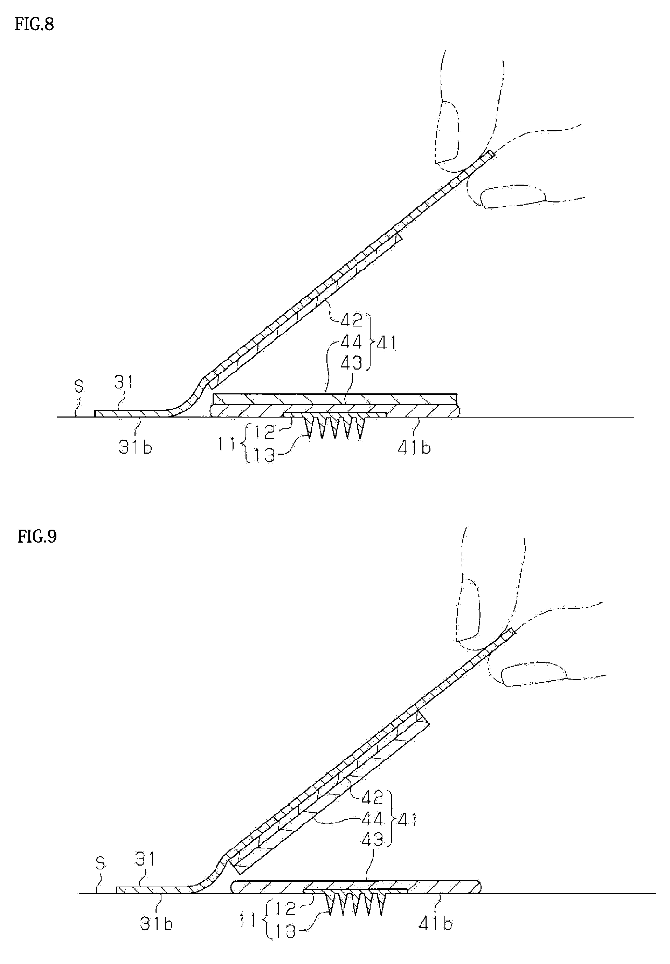

For example, as shown in Fig. 8, the adhesive strength of the cover contact section 42 to the support section 44 may be the peeling adhesive strength. In this configuration, when the cover 31 receives a force in the direction separating from the puncture target, the target contact surface 41b remains adhered to the skin of the puncture target, and adhesion between the cover contact section 42 and the support section 44 is released.

Further, for example as shown in Fig. 9, the adhesive strength of the target contact section 43 to the support section 44 may be the peeling adhesive strength. In this configuration, when the cover 31 receives a force in the direction separating from the puncture target, the target contact surface 41b remains adhered to the skin of the puncture target, and adhesion between the target contact section 43 and the support section 44 is released.

The outer peripheral contact surface 31b may not necessarily have adhesiveness to the puncture target. In this configuration as well, at least the effect similar to the above (1) can be obtained as long as the cover adhesive strength is smaller than the target adhesive strength in the adhering section 41.

In the adhering section 41, the cover adhesive strength may be equal to or larger than the target adhesive strength. For example, if the cover contact surface 41a is divided into a plurality of portions, the cover adhesive strength may be equal to or larger than the target adhesive strength. In this configuration, when the cover 31 is peeled from the microneedle 11, a force of peeling the cover 31 from the microneedle 11 is applied to each of the portions of the cover contact surface 41a. Accordingly, the cover 31 is peeled from the microneedle 11. In other words, the adhering section may have any configuration as long as the cover section contact surface 41a can be peeled from the cover section 31 when a portion of the target contact surface 41b located around the adhering surface 12b is adhered to the puncture target and the cover section 31 receives a force in the direction separating from the puncture target.

### Reference Signs List

10...microneedle unit, 11...microneedle, 12...base body, 12a...facing surface, 12b...adhering surface, 13...projection, 21...container, 21a...opening, 21b...housing space, 22...bottom, 23...cylindrical body, 24...flange section, 31...cover, 31a...flange facing section, 31b...outer peripheral contact surface, 31c...uneven area, 41...adhering section, 41a...cover contact surface, 41b...target contact surface, 42...cover contact section, 43...target contact section, 44...support section, S...skin

## Claims

1. A microneedle unit comprising:
a microneedle having a facing surface that faces a puncture target which is to be pierced by the microneedle and an adhering surface which is a surface opposite to the facing surface, the microneedle having one or more projections on the facing surface;
a recessed container that houses the microneedle, the recessed container including an opening that surrounds the microneedle;
a cover formed in a plate shape and is connected to the recessed container to close the opening, the cover extending from the adhering surface to an area surrounding the adhering surface; and
an adhering section located between the cover and the adhering surface to adhere the cover and the adhering surface, wherein
the recessed container is configured to be released from the cover in a state where the microneedle is adhered to the cover via the adhering section, when receiving a force in a direction separating from the cover, and
the adhering section includes a first contact surface that is in contact with the cover and a second contact surface that is in contact with the adhering surface and extends from the adhering surface to an area surrounding the adhering surface so that the second contact surface comes into contact with a portion of the puncture target located in an area surrounding the facing surface when the projections pierce the puncture target.

2. The microneedle unit according to claim 1, wherein the adhering section is configured such that, while the projections pierce the puncture target, when a portion of the second contact surface located in the area surrounding the adhering surface is adhered to the puncture target and the cover receives a force in the direction separating from the puncture target, the first contact surface is peeled from the cover.

3. The microneedle unit according to claim 1 or 2, wherein, in the adhering section, an adhesive strength of the second contact surface to the puncture target is larger than a force necessary for increasing a distance between the second contact surface and the cover.

4. The microneedle unit according to any one of claims 1 to 3, wherein the cover includes a third contact surface that comes into contact with a portion of the puncture target located in the area surrounding the adhering section when the projections pierce the puncture target,
at least part of the third contact surface has adhesiveness to the puncture target, and
an adhesive strength of the third contact surface to the puncture target is smaller than an adhesive strength of a portion of the second contact surface located in the area surrounding the adhering surface to the puncture target.

5. The microneedle unit according to any one of claims 1 to 4, wherein a forming material of the first contact surface and a forming material of the second contact surface are different from each other.

6. The microneedle unit according to any one of claims 1 to 5, wherein the cover includes a plurality of stepped portions on at least part of a portion that is in contact with the first contact surface.

7. The microneedle unit according to any one of claims 1 to 6, wherein the adhering section includes:
a first portion having the first contact surface;
a second portion having the second contact surface; and
a third portion disposed between the first portion and the second portion in a direction in which the microneedle and the puncture target face each other, the third portion having rigidity higher than the second portion.

8. The microneedle unit according to any one of claims 1 to 7, wherein the recessed container further includes a flange section that extends outward from an edge of the opening,
the cover further includes a flange facing section that faces the flange section, and
the flange section and the flange facing section are connected to each other.
